# EUROPEAN PATENT SPECIFICATION

(11) **EP 3 003 161 B1**
(45) Date of publication and mention of the grant of the patent: **12.01.2022**
(21) Application number: 14733531.9
(22) Date of filing: 28.05.2014
(51) Int. Cl.: A61B 8/14, A61B 8/00, A61B 8/08

(54) **METHOD FOR 3D ACQUISITION OF ULTRASOUND IMAGES**
VERFAHREN ZUR 3D-ERFASSUNG VON ULTRASCHALLBILDERN
PROCÉDÉ D'ACQUISITION EN 3D D'IMAGES ULTRASONORES

(30) Priority: 28.05.2013 EP 13169579
(43) Date of publication of application: 13.04.2016
(73) Proprietor: Universität Bern, 3012 Bern (CH); CAScination AG, 3014 Bern (CH)
(72) Inventor: RIBES, Delphine, CH-3013 Bern (CH); PETERHANS, Matthias, 3005 Bern (CH); WEBER, Stefan, CH-3067 Boll (CH)
(74) Representative: Schulz Junghans Patentanwälte PartGmbB
(86) International application number: PCT/EP2014/061106
(87) International publication number: WO 2014/191479

(56) References cited:
- WO-A1-2012/073164
- WO-A1-2013/055611
- US-A1- 2010 260 398
- US-A1- 2010 298 704
- US-A1- 2011 021 914
- GEE A ET AL: "Engineering a freehand 3D ultrasound system", PATTERN RECOGNITION LETTERS, ELSEVIER, AMSTERDAM, NL, vol. 24, no. 4-5, 1 February 2003 (2003-02-01), pages 757-777, XP004391215, ISSN: 0167-8655, DOI: 10.1016/S0167-8655(02)00180-0

## Description

The present invention relates to methods and systems used in ultrasound (US) imaging of biological soft tissues. More specifically, it relates to an US-acquisition protocol with an interactive real-time feedback to the user. The method allows fast and accurate imaging and localization of a specific anatomical structure of interest for and during, but not limited to, an image guided surgical or diagnostic intervention; particularly inner organs, such as the liver. Moreover, the invention ensures satisfactory image content for further image processing particularly for diagnosis, segmentation (e.g. the partitioning of a digital image into two or more regions corresponding to features of the imaged object such as vessels etc.) and registration.

### Background of the Invention

Three-dimensional (3D) ultrasound imaging is increasingly used and becoming a widespread practice in clinical environments due to its high potential of applications based on 3D representations of anatomical structures.

US 2010/0260398 A1 discloses a survey imaging mode implemented to provide a volume image of a relatively large survey area, wherein a target of interest is preferably identified within the survey area for use in a target imaging mode.

In conventional two-dimensional (2D) ultrasound imaging, the physician acquires a series of images on the region of interest while moving the ultrasound transducer by hand. Based on the content and the motion patterns used, he then performs a mental 3D reconstruction of the underlying anatomy. This mental process has various disadvantages: Quantitative information is lost (distances between anatomical structures, exact locations relative to other organs, etc.) and the resulting 3D information is dependent on and only known to the physician performing the scan.

Using 3D ultrasound (US) imaging and appropriate processing of the image data significantly helps eliminating the above stated disadvantages. Further benefits of 3D echography are as follows: In a 3D volume the spatial relationships among so called 2D slices are preserved, which allows offline examination of ultrasound images previously recorded by another physician. Using the so called any-plane slicing technique, image planes that cannot be acquired due to geometrical constraints imposed by other structures of the patient, can now be readily rendered. Further, the diagnostic task can be greatly improved by a volume visualization and an accurate volume estimation [1].

3D US-images are acquired using sophisticated ultrasound systems, which are described in various patent applications. There are mainly two approaches to obtain 3D data: one being the use of a 2D phased-array probe allowing for scanning of a volume of interest, and the other reconstructing a 3D volume from a series of 2D images acquired with a standard ultrasound which is moved over the region of interest.

The 2D phased-array probe technology employs a bi-dimensional array of piezoelectric elements. The volume is scanned by electronically steering the array elements. Dedicated 3D US-probes have been introduced for real-time 3D volume acquisition mainly in obstetric and cardiac imaging. Typical device examples are the Voluson^{®} 730 (GE Medical Systems) and the iU22^{®} (Philips Medical Systems, Bothell, WA, USA). Both systems aim to produce high-quality 3D US-images in all spatial directions (axial, lateral and elevational) with high acquisition rates of typically 40 volumes per seconds. Using this technique a completely filled 3D volume may be obtained.

The major disadvantage of this technique is that the field of view is limited by the size of the probe and that such probes are expensive and only available in high-end ultrasound devices. An alternative is to compound 3D sweeps from a series of 2D images as proposed in [2], [3]. This technique uses standard ultrasound probes (1-D piezo arrays) and different ways of scanning the region of interest (probe translation, probe rotation, free-hand scanning with probe position tracking). The ultrasound systems, probes and methods used for 3D images are state-of-the-art and are described in [4-10]. In the case of a so-called freehand ultrasound probe, an accurate freehand ultrasound calibration is required [11]. To ensure a homogenous and completely filled 3D volume the data acquisition should be performed with a homogeneous speed, an equal direction and angle, as described in [6]. To overcome the issue of acquisition artifacts sophisticated reconstruction and compounding algorithms have been described for example in US6012458A and [12].

An emerging application of 3D ultrasound is it's use for registration in navigated soft tissue surgery. Surgical navigation systems are used to guide physicians based in 3D image data, which is usually acquired in computer tomography (CT) or magnetic resonance imaging (MRI) before the surgery. As the soft tissues can deform and move in the time between imaging and surgery, additional intra-operative information data is required to warp (register) the pre-operative image data onto the patient being operated. Being real-time, non-invasive and commonly available, ultrasound imaging is a promising modality for acquiring such intra-operative data. As described above, especially 3D ultrasound imaging is well suited for acquiring such information on organ motion and deformation.

A common challenge with all 3D ultrasound acquisition techniques is the variance in image quality and the lack of measure indicating if the acquired data is sufficient for further image processing (such as diagnosis, segmentation and registration). The suitability of the image data for further processing depends on the image content, on the contrast between structures of interest and background, on the amount of artifacts present in the image, and on image homogeneity and density of volume scanning. The user performing the scans usually assesses all these factors once the scan is completed or after reviewing the results of further processing (e.g. in navigated surgery a 3D dataset is acquired, and registration is attempted and the registration result is analyzed). If the result of the scanning is insufficient, the entire acquisition process needs to be repeated - this is time-consuming and can be tedious as it is not sure whether the repetition of the scan leads to better results.

The available state of the art for guidance/feedback during ultrasound acquisition can be classified in
- Guidance for obtaining a desired image content
- Guidance for regular transducer motion
- Guidance of 3D imaging based on generic models of anatomy
as discussed in the following paragraphs:

### Guidance for obtaining a desired image content

In US 2012065510 the contents of an acquired B-Mode are compared with a model of the desired image in order to train the user for acquiring a desired view of an anatomical structure. For each acquired image a quality-of-fit measure to the desired target image is calculated and displayed to the user. The user then moves the ultrasound probe until an image with sufficient fit is obtained. This method provides a feedback on image quality of a 2D image but does not provide explicit instructions on how to improve image quality nor does it give indications on the usability of the image for further processing.

In US 2007016016 a general interactive assistant for imaging processes is presented. The assistant compares acquired images with a previously stored target image. If the similarity between the acquired image and the target image is not sufficient, the assistance tries to recommend actions for improving the image quality. As in the previously discussed patent, the image content of the two images is compared directly and with no consideration of further processing steps.

### Guidance for the regular transducer motion

In US005645066 a system for guiding free-hand 3D sweeps is described. The method trains the user to move at a regular speed over a desired region of interest in order to obtain a regularly spaced set of 2D ultrasound images, which are then compounded into a 3D volume. The feedback provided graphically shows the amount of filling of the image buffer user for compounding but does not investigate the image quality nor give any information on the 3D image volume being generated.

In US 2012108965 a method for coaching a user to perform correct probe motion for elastography imaging is described. By using sensors such as accelerometers inside the US probe, its motion is measured and compared to the desired motion pattern for elastography imaging. The system then provides a visual or auditory feedback to the operator in order to facilitate correct motion. This system is restricted to sensing the motion in space of the transducer and does not provide any feedback on the quality of image contents.

In US 20090036775 a method for visualizing scanning progress during laparoscopic ultrasound imaging is described. Based on a position measurement of the transducer, the acquired ultrasound images are displayed in 3D and the frames around the ultrasound images are highlighted if gaps exist in the scanning or if the scanning speed was too fast or too slow. This enables the user to re-scan missing areas and to make sure that a regular probe motion is achieved. As in the patents above, no feedback on the image content is provided.

### Guidance of 3D imaging based on generic models of anatomy

In EP1929956 a device for guiding acquisition of cardiac ultrasound is described. The system specifically displays the intersection of US image planes with a 3D-anatomical model in order to evaluate progress in data acquisition on the heart. The underlying analysis is therefore restricted to the geometric location of the image and does not include additional criteria regarding subsequent use of the image data.

In US 20080187193 an apparatus for forming a guide image for US scanning is presented. Based on a series of images acquired, a best-fitting 3D shape model is selected and displayed to the user. This 3D shape model then serves as guide image for subsequent imaging of the same structure. This enables efficient localization of important anatomical features and systematic scanning. The apparatus aims for guiding ultrasound scans towards certain target locations but is not focusing on obtaining 3D volume images of a desired, pre-defined quality.

Based on the description above, the problem underlying the present invention is to provide a method that eases the acquisition of a 3D ultrasound data set, i.e., a 3D model of a volume of interest of an object (e.g. body or body part, particularly organ, such as the liver, of a patient, and particularly allows for checking the quality of the acquired 3D model so that a specific further use of the acquired 3D model can be ensured.

This problem is solved by a method having the features of claim 1.

Preferred embodiments are stated in the corresponding sub claims, respectively, and are described below.

According to claim 1 the method according to the invention comprises the steps of: providing a pre-acquired 3D image or model (i.e. a corresponding data set) of an object (e.g. of a body or body part of a person/patient, for instance an organ such as the liver), displaying said pre-acquired image on a display (e.g. a graphical user interface (GUI) of a computer), selecting a volume of interest of the object (i.e. a certain volume of the object shall be examined) in said pre-acquired image (e.g. on the display with help of a GUI of a computer connected to the display), and particularly adjusting the spatial position of said volume of interest with respect to (an e.g. local coordinate system of) said pre-acquired image by positioning an ultrasound (US) probe with respect to the object (e.g. on a body of the patient) accordingly, particularly visualizing the current spatial position of said volume of interest (also denoted as VOI) on said display with respect to said pre-acquired image, particularly in real-time, as well as particularly displaying a current (e.g. 2D) ultrasound image on said display in real-time acquired in the volume of interest by means of the ultrasound probe, wherein particularly the visualization of the volume of interest is overlaid on the displayed pre-acquired 3D image, and particularly updating the visualization of the volume of interest on said display using the current spatial position of said ultrasound probe, which current spatial position of the ultrasound probe (e.g. in a so called room-fixed, patient-fixed or camera coordinate system) is particularly determined using a tracking system.

Now, when the spatial position of the volume of interest is selected or adjusted as intended (then the VOI remains static in the sense that it is not adjusted anymore), the acquisition (recording) of ultrasound images in said volume of interest in order to generate a 3D model (i.e. a corresponding data set representing the model or alternatively a 3D ultrasound image) of said object in said volume of interest is triggered, wherein said triggering is particularly performed by means of said ultrasound probe, particularly by means of a specific movement of or a defined gesture with the ultrasound probe with respect to the object (e.g. over the volume of interest), or a certain pre-defined position of the ultrasound probe, or by not moving the ultrasound probe for a pre-defined time period, or even automatically; and acquiring (particularly intraoperatively) a plurality of ultrasound images by means of said ultrasound probe in the volume of interest for generating said 3D model while moving the ultrasound probe over said object along or over the volume of interest (e.g. the ultrasound probe is preferably moved such on/over the object that images can be acquired in the VOI of the object), wherein the current image is particularly displayed in real-time on said display, wherein particularly the current image is displayed two-dimensionally on said display and/or three-dimensionally (e.g. in a 3D viewer shown on the display), wherein said three-dimensionally displayed current ultrasound image is particularly overlaid on the displayed pre-acquired image, and automatically determining if the current ultrasound image has at least a pixel in the volume of interest, wherein in case the current image has no pixel in the volume of interest, the current image is automatically discarded (i.e. not compounded into 3D model/ultrasound image), otherwise (i.e. when the image has a pixel or voxel in the VOI) the current ultrasound image is segmented and compounded into said 3D model to be generated which is displayed in real-time on the display and particularly overlaid on the displayed pre-acquired image, wherein particularly in case a new current ultrasound image is compounded into the 3D model, the displayed 3D model on the display is updated, and automatically determining a quality measure for the 3D model to be generated upon said acquiring of said ultrasound images, wherein said acquiring of said ultrasound images is ended once said quality measure has reached a pre-defined level, wherein particularly said quality measure is at least one of: the number of single (2D) ultrasound images scanned within the volume of interest, the (3D) density of the acquired ultrasound images within the volume of interest (e.g. the ratio between the scanned pixels or voxels and the number of pixels or voxels of the VOI, i.e., the VOIs volume), the number and/or distribution of specific image features, particularly the number of segmented anatomic structures in the volume of interest (e.g. such as tumors, vessels etc.), and the time that is used for scanning the ultrasound images. Other criteria may also be applied.

E.g. the acquisition is stopped in case the number of acquired (2D) ultrasound images exceeds a pre-defined number, or the acquisition is stopped in case the density of 2D ultrasound images in the VOI exceeds a pre-defined density value, or the acquisition is stopped in case a certain number and/or distribution of specific image features has been detected, or the acquisition is stopped after a pre-defined time period (assuming that the VOI was sufficiently sampled in this time period). After acquisition of the ultrasound images, the generated 3D model is registered to the pre-acquired, 3D image.

Thus, in particular, the present method allows for interactively acquiring ultrasound images with the purpose of image registration, i.e. a fusion between image modalities. Due to such a fusion, images which can be acquired during a treatment can be enhanced using much more detailed information acquired outside the treatment room (e.g. ultrasound images with a lower number of small vessels detected and lower contrast during the treatment are fused with high-resolution pre-operative CT or MRI). Particularly, the present invention aims at building an image acquisition framework, which does not merely aim to acquire high resolution images of the patient, but rather aims at acquiring technical information, which enables said fusion. Preferably, using patient-specific, a-priori knowledge from pre-operative data (usually from other modalities with better level of detail than ultrasound), the user is guided to acquire images/features required to perform the registration between the pre-acquired data and the current data acquired.

According to a preferred embodiment, said provided pre-acquired 3D image is acquired in a first session, whereas said plurality of ultrasound images are acquired in a separate second session that is conducted at a later time. The first session can be hours/days/weeks before the second session, e.g. surgery/intervention. Particularly the period of time between the two sessions is at least 1 hour, at least 12 hours, at least a day, or at least a week.

According to a further embodiment of the method according to the invention, said provided pre-acquired 3D image is acquired by using an imaging method other than ultrasound.

According to a further embodiment of the method according to the invention said quality measure is a criterion based on patient-specific data from said pre-acquired 3D image.

According to a further embodiment of the method according to the invention, said number and/or distribution is selected depending on the patient-specific anatomy in the volume of interest.

According to a further embodiment of the present invention, a user acquiring said plurality of ultrasound images is guided to move the ultrasound probe to a location where image features are expected based on the pre-acquired 3D image, particularly so as to provide a sufficient dataset for registering the generated 3D model to the pre-acquired 3D image.

According to an embodiment of the present invention, the VOI is not necessarily navigated with the ultrasound probe, but defined by placing the US probe at a certain location.

Further, "overlaying" an ultrasound (US) image on the pre-acquired 3D image or model particularly means that said US image or at least a portion of the US image is displayed at a position in the pre-acquired image such that a content or a feature of the US image is aligned or matches a corresponding content or feature of the pre-acquired image. The US image may thereby also supplement features or a content of the pre-acquired image or vice versa. Further, the US image may thereby cover portions of the pre-acquired 3D image. In case of a VOI, overlaying particularly means that a visualization of the VOI (e.g. a 3D box etc) is displayed in the pre-acquired 3D image, particularly at the proper position corresponding for instance to the position of the ultrasound probe in the room-fixed (or patient-fixed or camera) coordinate system.

Thus, the invention described herein guides the user for acquiring a 3D ultrasound model/dataset, which fulfills the requirements for further processing. Guidance is provided through an online, real-time analysis and display of the acquired 3D model and through a quantitative evaluation of image quality/content with regard to the subsequent processing requirements.

In order to accomplish said adjusting of the spatial position of said volume of interest with respect to said pre-acquired image, as well as overlaying the visualization of the volume of interest on the displayed pre-acquired image, as well as overlaying said three-dimensionally displayed current ultrasound image on the displayed pre-acquired image, or in order to accomplish checking whether the current ultrasound image has at least a pixel in the volume of interest, as well as overlaying the 3D model on the displayed pre-acquired image, an initial registration (yielding at least a rough alignment), is preferably performed. This allows one to (at least approximately) display US images, VOIs etc. in or on the pre-acquired 3D image at the correct position so that features or content of the displayed US images align with corresponding features or content of the pre-acquired 3D image or model.

Particularly the initial registration can be a landmark-based registration, where the user selects e.g. four points in the pre-acquired 3D image (e.g. a virtual liver model) and then touches them with a tracked tool (in order to acquire the points in the camera, patient-fixed or room-fixed coordinate system). A suitable algorithm then automatically calculates the registration transform.

Alternatively, or in combination, an ultrasound-based initial registration can be employed, where the user selects a point in the pre-acquired 3D image (e.g. virtual liver surface), where he would like to place the ultrasound probe. Then, the expected ultrasound image at that location is simulated using the pre-acquired 3D image and the user uses the calibrated ultrasound probe on the patient (object) to acquire the same image in the patient (hence in the camera, patient-fixed or room-fixed coordinate system). Based on the simulated virtual image and the acquired real image, the initial registration transform is automatically calculated. In this regard, a calibrated ultrasound probe is an ultrasound probe where a relation between the position of the acquired image in the room-fixed (or patient-fixed, or camera) coordinate system and the position of (the position sensor of) the ultrasound probe is known, so that knowing the position of the ultrasound probe means knowing the position of the acquired ultrasound image in the room-fixed (or patient-fixed or camera) coordinate system.

In a preferred embodiment of the method according to the invention, the generated 3D model is automatically registered to the pre-acquired, particularly preoperatively acquired, 3D image, particularly by matching one or several features of the generated 3D model, whose coordinates in the room-fixed (or patient-fixed or camera) coordinate system are acquired with help of tracking the ultrasound probe, with one or several corresponding features of the pre-acquired 3D image, and particularly by automatically determining a registration transform between the coordinate system of the pre-acquired 3D image and the room-fixed (or patient-fixed or camera) coordinate system of the ultrasound probe using the coordinates of said features and said corresponding features in the respective coordinate systems.

In other words, in the context of the present method according to the invention, the user defines a volume of interest (VOI), where the registration shall be performed. Definition of the VOI is either performed by clicking on the virtual model (i.e. the pre-acquired image) or by interactively placing the VOI using gestures with the ultrasound probe as described above (if gestures are used, the initial registration or alignment described above is used to display the position of the probe on the virtual model (i.e., the virtual model is mapped into the camera or room-fixed or patient-fixed coordinate system). The VOI can also be defined based on the landmarks selected in the initial registration described above (around the landmarks). Once the VOI is defined, acquisition is started either automatically or by a gesture. The feedback loop guides the acquisition of ultrasound images in the box (VOI) as described above. Once enough data is acquired, the registration is calculated.

Once this ultrasound-based registration is completed, the position of the pre-acquired 3D image, i.e., of the virtual 3D model, relative to the room-fixed (or patient-fixed or camera) coordinate system is known. Therefore, a tool, such as a surgical tool, whose position is tracked in the room-fixed (or patient-fixed or camera) coordinate system can be displayed on the pre-acquired 3D image (virtual model).

According to a preferred embodiment of the method according to the present invention, the volume of interest is pre-defined concerning its spatial dimensions in units of voxels (height, width and depth) and is further predefined or selected with respect to certain features or characteristics, particularly with respect to its spatial resolution, density of the detected or segmented structures, and/or homogeneity (i.e. its spatial density, wherein in this sense VOIs in pre-acquired images are preferred which are evenly sampled throughout) or number of artefacts (i.e. VOIs are preferred having a number of artefacts which is as small as possible, preferably no artefacts, as well as a low noise level).

Further, in a preferred embodiment of the method according to the present invention, an artefact detection is automatically conducted for the non-discarded current ultrasound image, particularly using at least one filter algorithm, particularly the Hough transformation and/or low pass filtering, wherein particularly in case an artefact is detected in the current ultrasound image this current ultrasound image is discarded, and wherein particularly an artefact probability is calculated based on patient-specific features of the pre-acquired 3D image.

Further, in a preferred embodiment of the method according to the present invention said segmentation of the individual current ultrasound image is automatically conducted using at least one (e.g. deterministic) algorithm providing segmentation of specific anatomic structures of the object in the volume of interest, particularly vessels, tumors, organ boundaries, bile ducts, and/or other anatomy, wherein particularly said algorithm is selected depending on patient-specific features of the pre-acquired 3D image.

Further, in a preferred embodiment of the method according to the present invention said segmentation of the individual current ultrasound image is automatically conducted using a probabilistic assessment of image features, particularly such as organ boundaries, organ parenchyma, and/or vessel systems, wherein said probabilistic assessment preferably uses patient-specific features of the pre-acquired 3D image.

Further, in a preferred embodiment of the method according to the present invention, the US-volume reconstruction algorithm applies two parallel process steps, one for the segmentation of information from different 2D US images and one for testing for image artefacts, either by directly using the 2D US image content or based on enhancement results, i.e. detected features or structures of the US image (e.g. after segmentation of the image). In other words, said artefact detection and said segmentation is preferably conducted in parallel, wherein particularly said artefact detection directly uses the individual content of the current ultrasound image or a detected content of said current ultrasound, and wherein particularly the respective algorithms iteratively interact with each other.

Preferably, the detected image features in the individual current 2D ultrasound images (having no artefacts) are then automatically combined to a 3D volume data set (which is also denoted as compounding) representing the 3D model that is successively generated upon acquisition of the series of (current) 2D ultrasound images.

Further, in a preferred embodiment of the method according to the present invention, during positioning the ultrasound probe for said initial adjusting of the spatial position of the volume of interest and/or upon moving of the ultrasound probe during said acquisition of the plurality of ultrasound images, guiding information is displayed on said display and/or acoustically provided to the user, particularly verbally, in order to assist and/or guide the user when positioning and/or moving said US probe.

Preferably, said guiding information is provided through feedback based on said pre-acquired 3D image and acquired features of the 3D model.

Preferably, the ultrasound probe is tracked by deriving the spatial image coordinates (i.e. in the room-fixed, patient-fixed or camera coordinate system) using a coordinate measurement system based on an optical, electromechanical or mechanical measurement principle and/or by deriving relative image coordinates by analyzing the relative shift of image features in subsequent images.

Furthermore, preferably, said guiding information comprises a visualization of at least one or several cubical grids on said display, wherein particularly specific colors represent defined tissue structures and/or anatomic structures. Further, preferably, said grid or grids are displayed on the pre-acquired 3D image.

Further, in a preferred embodiment of the method according to the present invention, particularly upon said segmentation, missing information in the current ultrasound image is automatically interpolated based on a-priori information or using a priori-information about the object (e.g. organ) or patient-specific features from the pre-acquired 3D image. Further, upon said segmentation, missing information in the current ultrasound image (401, 402) can be interpolated using cohort specific and/or statistical information about the distribution of vascular structures, geometric shapes of the anatomic structures of interest in the object, object parts or lesions, and/or other known anatomical structures.

Preferably, the volume of interest is chosen such that it contains sufficient image information to allow for further processing towards diagnosis, visualization, segmentation and/or registration.

Further, in a preferred embodiment of the method according to the present invention, the generated 3D model is e.g. automatically aligned with the pre-acquired 3D image, that is particularly based on an imaging method other than ultrasound and particularly based on a different coordinate system compared to the 3D model, so as to display the current level of progress of the 3D model generation, particularly with respect to previously acquired or dynamically refreshed information content, particularly with respect to parameters such as homogeneity (see above) and/or resolution.

Further, preferably, the visualization of the 3D model on the display uses user-defined static or dynamic color mappings, particularly indicating anatomic structures currently detected and analyzed.

Further, in a preferred embodiment of the method according to the present invention, the successful completion of the ultrasound image acquisition process is signalled to the user, particularly acoustically via a speaker and/or graphically via said display.

Preferably, the pre-acquired 3D image is an ultrasound, computer tomography, or magnetic resonance image.

Furthermore, a system is disclosed which is particularly designed to conduct the method according the invention, wherein said system comprises: an ultrasound probe connected to a data processing system, which data processing system particularly comprises a control unit for control of said ultrasound probe, a computing means (e.g. a computer, e.g. such as a PC or work station) for the acquisition and analysis of US images, and a display connected to said computer for displaying information, particularly US images and pre-acquired images as well as information for the user (e.g. guiding information). Further, the system comprises a tracking system for tracking the spatial position of the ultrasound probe (e.g. with respect to a room-fixed, patient-fixed or camera coordinate system), the tracking system comprising one or several position sensors arranged on or integrated into the ultrasound probe for detecting the spatial position of the ultrasound probe in said coordinate system, wherein said tracking system (also denoted as coordinate measuring system) is particularly designed to sense the position of the ultrasound probe optically, electromechanically, or mechanically, i.e., said tracking system is based on an optical, electromechanical or mechanical measurement principle for position tracking of the ultrasound probe.

Preferably, the tracking system comprises a tracking device, such as a camera, particularly a stereo camera, being designed to detect and track the position of the position sensor(s) in a camera coordinate system that rests with the camera (or tracking device). Such a coordinate system may also denoted as room-fixed or patient-fixed coordinate system since the tracking device usually rests with respect to the room in which the patient is located or with respect to the patient.

Preferably, the data processing system is designed to automatically check if a current ultrasound image of an object acquired with the ultrasound probe has at least a pixel in a pre-selected volume of interest of a pre-acquired 3D image of the object, wherein in case the current image has no pixel in the volume of interest, the data processing system is designed to discard the current image, wherein otherwise (i.e. when the image has a pixel/voxel in the VOI) the data processing system is designed to automatically segment the current ultrasound image and to compound it into a 3D model, and wherein the data processing system is designed to determine a quality measure for the 3D model to be generated, particularly upon acquisition of ultrasound images with the ultrasound probe, wherein the data processing system is designed to end the acquisition of ultrasound images for the 3D model once said quality measure has reached a pre-defined level or dynamically defined level, wherein particularly said quality measure is at least one of: the number of single ultrasound images) scanned within the volume of interest, the density of the acquired ultrasound images within the volume of interest, the number and/or distribution of specific image features, particularly the number of segmented anatomic structures in the volume of interest or particularly a patient-specific number of expected features, and the time needed for the acquisition of the ultrasound images (see also above).

Further, the data processing system is particularly designed to automatically register the generated 3D model to the pre-acquired 3D image, or vice versa (see also above).

The system may further comprise a speaker for providing a user with acoustic, particularly verbal, information (e.g. guiding information, see also above).

The system can be further characterized by the features of the methods according to invention described herein.

Further, a computer program is disclosed comprising program commands which cause a computer (e.g. said data processing system or said computer of the data processing system) to conduct the method according to the invention (e.g. according to claim 1) when the computer program is loaded into the computer or executed by the computer. Here, particularly, the pre-acquired 3D image, the current (2D) ultrasound images acquired with the ultrasound probe, and/or the VOI are fed to the computer program as an input.

Particularly, a computer program is provided comprising program commands which cause a computer (e.g. said data processing system or said computer of the data processing system) to check if a current ultrasound image has at least a pixel in a volume of interest, wherein in case the current image has no pixel in the volume of interest, the current image is discarded, wherein otherwise the current ultrasound image is segmented and compounded into a 3D model to be generated which is particularly displayed in real-time on a display (e.g. connected to said computer) and particularly overlaid on a displayed pre-acquired image, wherein particularly in case a new current ultrasound image is compounded into the 3D model, the displayed 3D model on the display is updated, and to determine a quality measure for the 3D model to be generated, particularly upon acquisition of said ultrasound images, wherein an acquisition of said ultrasound images is ended once said quality measure has reached a pre-defined level, wherein particularly said quality measure is at least one of: the number of single ultrasound images scanned within the volume of interest, the density of the acquired ultrasound images within the volume of interest, the number and/or distribution of specific image features, particularly the number of segmented anatomic structures in the volume of interest, and the time needed for scanning the ultrasound images (see also above).

Further, another aspect of the present invention is a method for the real-time generation and visualization of guiding information for a user to assist in the localization and identification of a suitable position of a volume of interest for placement of an ultrasound probe on an organ's surface.

In this regard, tracking of the ultrasound probe is preferably enabled by deriving the absolute spatial image coordinates using a coordinate measurement system based on an optical, electromechanical or mechanical measurement principle and/or by deriving relative image coordinates by analyzing the relative shift of image features in subsequent images.

Further, in this regard, guiding information for the user preferably comprises virtual visualizations of cubical grids in a display of a graphic user interface with specific colors representing defined tissue structures, particularly anatomic structures. Further, in this regard, the guiding information for the user is preferably acoustic or verbal (e.g. recorded spoken words or artificial voice).

According to yet another aspect of the present invention, a registration method is provided to align acquired 3D ultrasound images with pre-acquired 3D image data sets to display a current level of progress of the 3D volume image acquisition with respect to previously acquired or dynamically refreshed information content, particularly with respect to but not limited to parameters such as homogeneity (see above) and/or resolution.

In this regard, the visualization of the 3D ultrasound image data sets preferably employs specific, user-defined static or dynamic color mappings indicating anatomic structures currently detected and analyzed.

Further, in this regard, the successful completion of the image acquisition process is preferably signalled to the user acoustically and/or graphically via means of a GUI and/or an acoustic interface, particularly a speaker.

Further, in this regard, the pre-acquired images are preferably ultrasound, CT- or MR-images, particularly of heterogeneous quality and image content.

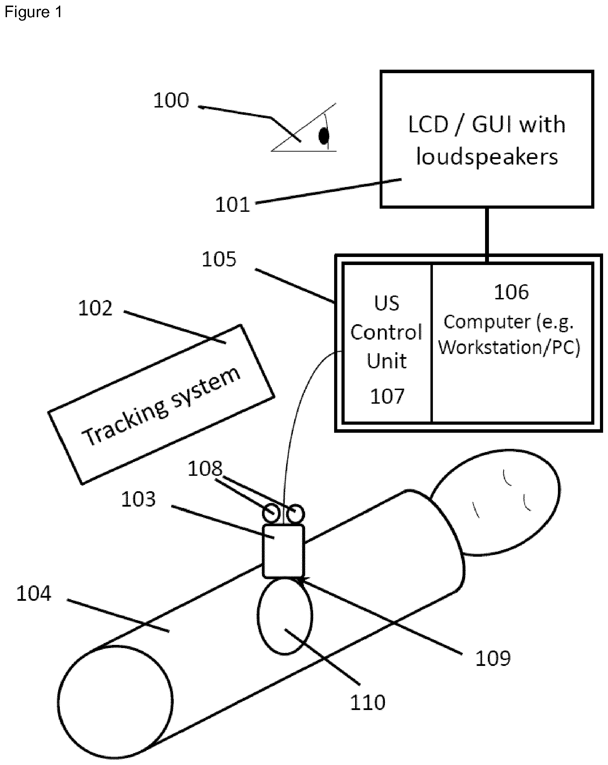

Further features and advantages of the invention shall be described by means of detailed descriptions of embodiments with reference to the Figures, wherein
- Fig. 1: shows a typical embodiment for 3D ultrasound (US) image acquisition;
- Fig. 2: illustrates in a schematic diagram the initialization of the US image acquisition process;
- Fig. 3A and 3B: show an anatomical structure and the visualization of the volume of interest (VOI). Figure 3B shows the visualization of the VOI together with a 2D US image; In figure 3A, the graphical representation of VOI and ultrasound image are overlaid onto the 3D anatomy;

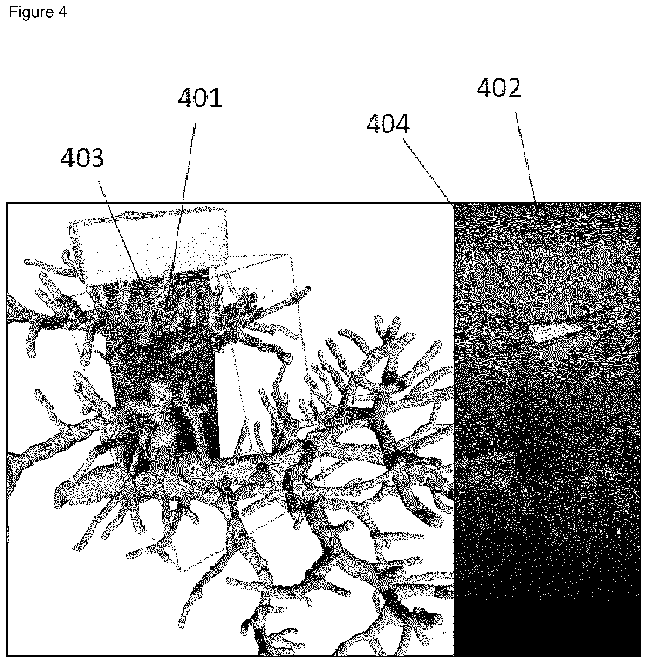

- Fig. 4: shows the visualization during 3D ultrasound image acquisition;
- Fig. 5A: illustrates the US image acquisition process which includes artifact detection and image segmentation;
- Fig. 5B: shows a typical picture of an artefact in an US image; and

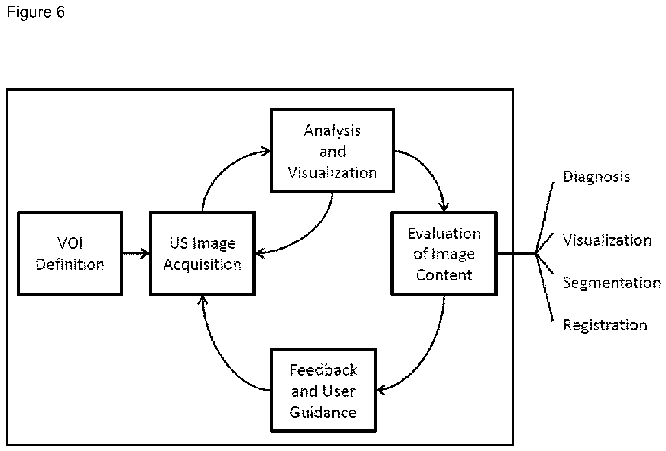

- Fig. 6: shows the acquisition algorithm with the real-time feedback to guide the user for acquiring suitable image data for further image processing.

Particularly, the method according to the present invention serves for optimizing the acquisition of 3D US images with the principle aim of improving the real-time registration of US images with pre-acquired (e.g. 3D) images, particularly from US, CT and/or MR. By employing an online or real-time 3D image analysis loop and a real-time feedback during the acquisition of US-images, the system/method aims to ensure suitable image content of 3D US images/models for further data processing, i.e. diagnosis, visualization, segmentation and registration.

The invention is particularly described in relation to image registration for navigated soft tissue surgery but is however not limited to this application.

### System Setup

According to an exemplary embodiment, the method according to the invention particularly uses the following components: A 3D or 2D ultrasound (US) probe 103 connected to a data processing system or unit 105 comprising a control unit 107 for controlling the US probe 103 and a computer (workstation or PC) 106 with a graphic user interface (GUI) 101 for displaying of image- and other relevant user information. The display 101 may consist of a screen-display (LCD or similar) and other means of graphical and/or visual display of information to the user 100. Also, speakers may be attached to the computer 106 or GUI 101.

The US probe 103 is tracked by means of an e.g. commercially available tracking system 102. The US probe 103 is calibrated and has attached or integrated passive or active tracking sensors or reflectors 108 also denoted as position sensors 108. Particularly, feedback and guidance for the acquisition of suitable image content is based on geometrical information of the acquired image relative to the desired volume of interest as well as on measures of the obtained information content in 3D. Such measures are derived from the segmentation of the acquired images and can be provided to the user as qualitative 3D display or by quantitative indicators of quality. By providing online feedback during 3D image acquisition, the operator is guided to move the US probe to the missing scanning location, to adjust imaging parameters correctly and finally to ensure that sufficient data is acquired for subsequent processing. By controlling image quality during the acquisition process, tedious repetition of the entire imaging process can be avoided.

### Adjustment, Visualization and Selection VOI

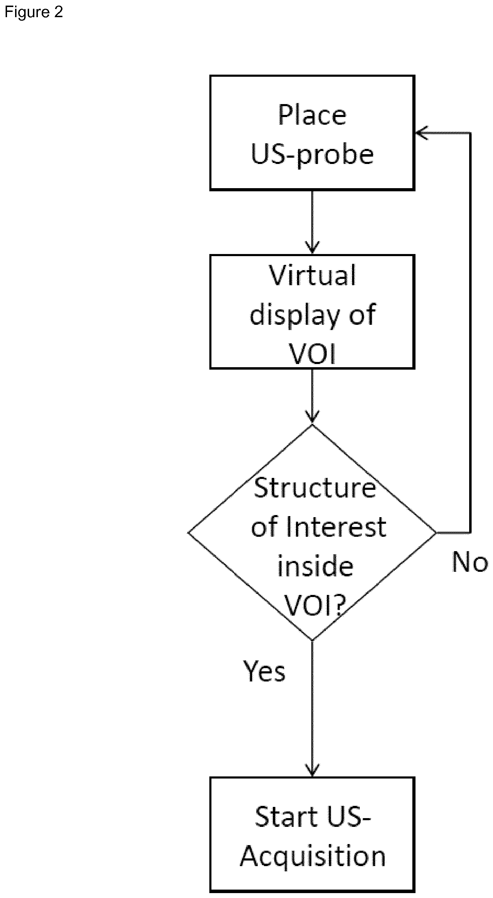

The user 100 selects the so-called volume of interest VOI 301 in a pre-acquired image from ultrasound- (US), computed tomography- (CT) or magnetic resonance (MR) imaging, which is displayed in the display 101 of the GUI of the system. Prior to the selection of the VOI 301 an initial registration may be performed based on one or several landmark points in order to roughly align the pre-acquired anatomical image or model to the tracking coordinate system (room-fixed coordinate system). The position of the VOI 301 is then adjusted by the user placing the US probe 103 on the surface of the organ 110 of interest. The current VOI 301 is displayed in the GUI 101 and updated based on real-time tracking information. The user 100 thereby receives real-time, visual feedback on the GUI 101 allowing him to interactively select the appropriate VOI 301, i.e. the anatomical structure of interest 302. The algorithm for adjustment of the VOI 301 is illustrated in Fig. 2.

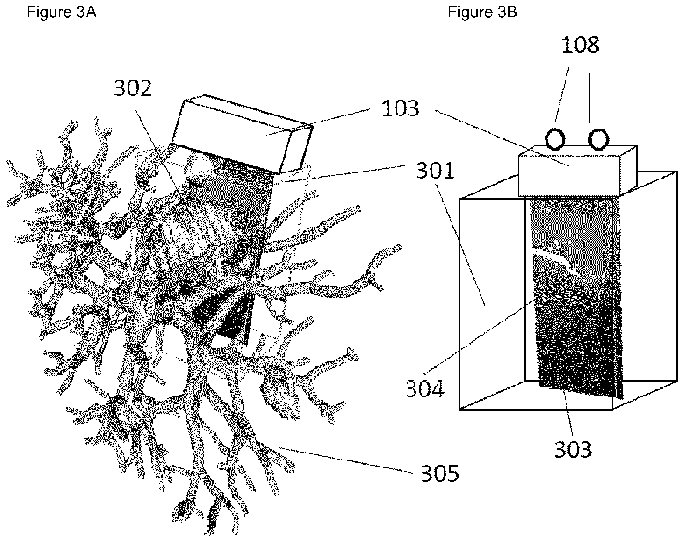

During the adjustment phase, the VOI 301 is visualized as a virtual cubical grid with specifically colored lines together with the first US-image (Fig. 3B) on the GUI 101. The VOI 301 is placed below the virtual model of the tracked US probe 103 and VOI's 301 position is updated with the motion of the probe 103. The overlay of the virtual VOI 301 onto the pre-acquired image or model 305 of the anatomy of interest (Fig 3A) enables the user to visually analyze the location and orientation of the selected VOI 301, particularly whether the anatomical structure of interest being inside the VOI 301. The user 100 moves the US probe 103 over the surface of the organ 110 until the spatial placement of the VOI 301 is satisfactory.

Once correct placement of the VOI 301 is achieved, the VOI 301 is selected holding the probe 103 still on the desired location or by other interaction means within the reach of the user 100 (e.g. by pressing a confirmation button on the GUI 101, or by using a voice command).

The size of the VOI 301 is determined by the following parameters: the length of the US probe 103, the image depth and the expected anatomical structure of interest. For example, the VOI 301 of an inner organ, such as the liver, typically entails a branch of a vessel system, a functional segment, a tumor or an accumulation of tumors, organ boundaries, bile ducts or/and organ parenchyma. Further, the structure may also be a probabilistic representation of expected features such as organ boundaries (probability of organ boundary being within a certain region). Typical VOI 301 dimensions are approx. 40 mm (length) x 80 mm (width) x 90 mm (depth). Fig. 3A and 3B show a typical VOI 301.

### Acquisition of ultrasound images in the VOI

Once the VOI 301 selection is completed, 3D data acquisition starts. If the user places the probe 103 for imaging a region outside the VOI 301 during the image acquisition process, he is informed acoustically and/or visually via the GUI 101. The information may displayed by a specific symbol / pictogram, such as a colored arrow or hand or/and it may be encoded into a sound (e.g. by means of frequency or amplitude modulation, beep length). The acoustic information may also comprise verbal instructions to the user 100 given by means of one or more speakers.

The individual acquired (e.g. 2D) current US images 401, 402 are displayed in real-time on the GUI 101. In this way the user 100 can interactively, visually check if the anatomical structures of interest are visible in the US image. The visualization can be provided as standard 2D ultrasound image 402 and also in a 3D viewer 401. The 3D viewer can either display only the ultrasound image and it's location within the VOI 301 (similar to Fig. 3B) or it can superimpose the acquired image with the corresponding 3D information from pre-acquired images (similar to Fig. 3A).

### Online image quality check, segmentation and compounding

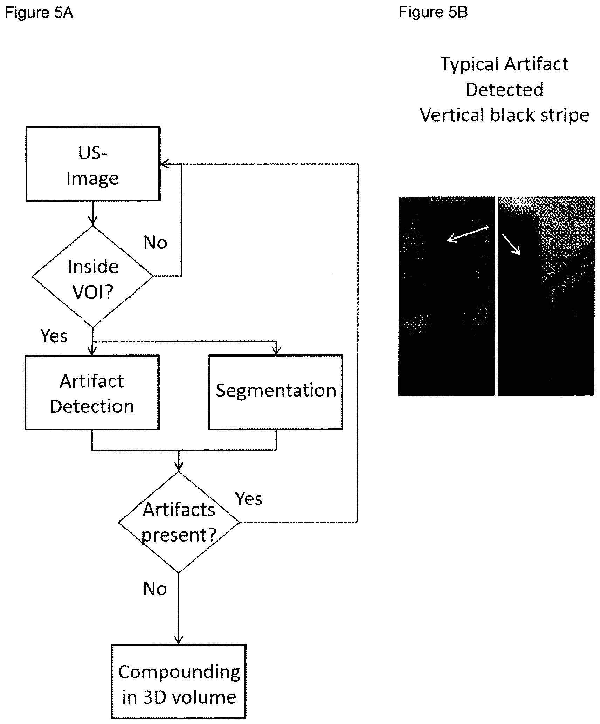

During the image acquisition an automatic online image quality check and analysis is performed. An example for the image evaluation algorithm is illustrated in Fig. 5. The algorithm takes an acquired (current) US-image and checks whether the location of the image is inside the selected VOI 301. If the image is not inside the selected VOI 301, the next acquired (current) image is analyzed. This automatic process uses the spatial information from the tracking system 102 and the tracking sensor(s) 108 attached to the US probe 103. From the tracking information and the US calibration transform (i.e. a transformation which links the position of the US probe 103, e.g. the position of a position sensor attached or integrated into the probe 103, to the position of the US image generated with the probe, so that knowing the position of the US probe 103 in the room-fixed, patient-fixed or camera coordinate system means knowing the position of the US image in this coordinate system), the 3D spatial position of the US image is computed and compared with the 3D spatial position of the VOI 301. If no pixel of the US image is positioned inside the VOI 301, the US image is considered as being outside of the VOI 301. Otherwise, the image is considered as valid and used for further processing, which includes artefact removal and segmentation. The artefact removal process detects US-specific artefacts such as large black stripes (Fig. 5B) in the image. These may originate from an insufficient contact between the active sensing area of the US probe 109 and the biological tissue / organ of the patient 110 or from rigid structures reflecting the complete US signal. By using methods such as the Hough transformation, low pass filtering or intensity analysis along vertical lines of the image, black stripes are automatically detected.

In parallel to the artefact detection process, the image is segmented and buffered until the artifact detection is completed (see Fig. 5). If there are no artefacts present, the segmented image is retained and compounded in the 3D US image/model. Segmentation automatically detects structures of interests in the image (typically vessels, tumors or organ boundaries) and displays them as an overlay onto the 2D image 404. If a new US image is compounded into the 3D US volume, the 3D information 403 on the GUI 101 is updated and displayed to the user 100. By displaying the results of the analysis in real-time on the 2D image, the user 100 can interactively determine whether the segmentation algorithm successfully detects relevant information on the current image. By updating the 3D visualization with recently acquired data, the user 100 further obtains feedback on the overall acquisition process and can judge if there are locations where information is missing and finally determine if a sufficient representation of the anatomy of interest was acquired.

In addition to the information on the acquired image content, the GUI 101 can also display all the acquired image planes and thereby provide a visual feedback on the filling of the VOI 301 with ultrasound images. This enables the user 100 to see locations where no image data was acquired and to interactively place the ultrasound probe 103 on these locations.

The algorithms used for segmentation are chosen according to the anatomical structure of interest. Typical examples are algorithms for vessel detection and for organ surface detection. A vast range of US segmentation algorithms are available in the state of the art [16].

The methods for image compounding employed are well known and are as follows, but not limited to pixel nearest-neighbor (PNN), voxel nearest-neighbor (VNN), distance-weighted (DW) interpolation, non-rigid registration, radial basis functions (RBF) interpolation and Rayleigh model for intensity distribution. They are described in literature [12].

### Quantitative measure of information content

In addition to the visual feedback provided to the user, processes running in parallel to the image acquisition perform automatic quantitative analysis of the US image data. Such measures ensure that the image content is suitable for further processing and provide additional real-time feedback to the user 100. Typical quality measures in the context of registration for navigated soft tissue surgery include percentage of the VOI 301, which was scanned with the ultrasound probe 103 (e.g. 10% of the voxels in the VOI where scanned) or the amount of anatomical data detected (e.g. number of segmented vessel/tumor/boundary) voxels. As the expected/required image content is known from the pre-acquired volumetric image data, the measure of currently acquired information content can be put in relation to the required data for further processing. In the case of navigated liver surgery, the system aims to detect a branch of a vessel system, which is then used for registration. The dimensions of the vessel system (and the expected number of vessel pixels) are known from pre-operative imaging and the feedback loop can therefore the percentage vessels detected with intra-operative ultrasound. A similar amount of data in both, the pre- and intra-operative dataset is expected to lead to robust and accurate registration.

### Feedback Loop

Figure 5 depicts the complete 3D image acquisition incorporating all the components described above. The process starts with an interactive VOI 301 definition using a virtual display of the planned VOI 301, which is connected to the navigated ultrasound probe 103.

Once a VOI 301 is defined, the system enters a loop where each newly acquired image is analyzed to determine if it depicts structures within the VOI 301 and contains no artefacts. If the image is outside the VOI 301 or contains an artefact, the algorithm returns to image acquisition. If not, the image is segmented and compounded and the resulting data is displayed to the user 100 on the GUI 101. Based on the visual feedback on the GUI 101 and the quantitative measures of information content, a criterion for stopping the US acquisition is evaluated. The criterion for stopping the image acquisition is defined prior or during to the acquisition process and varies with the organ or tissue 110 to be analyzed. In general there are three fundamental options of definition for the criterion: (a) visual definition by user, (b) a static criterion based on the US data acquired (e.g. number of valid images acquired, percentage of volume filled, percentage of segmented voxels), and (c) a dynamic criterion based on the expected image content (e.g. prediction of number of intra-operative vessels pixels expected based on the pre-operative image data and VOI selection). Hence either the user 100 or the acquisition algorithm decides whether the acquired image content is sufficient for the desired application (diagnosis, visualization, segmentation, registration) or if additional images need to be acquired. If sufficient data is available, acquisition is stopped, otherwise feedback on the required additional image content is provided to the user 100.

The feedback to the user 100 includes visual or auditory instruction about the necessary actions (e.g. probe motion to other area of the VOI 301, search of anatomical structures, changes in imaging parameters) for obtaining the required image quality. Based on this feedback, the user acquires a next image and the feedback loop starts from the beginning.

### References

[1] R. San Jose-Estepar, M. Martin-Fernandez, P. P. Caballero-Martínez, C. Alberola-Lopez, and J. Ruiz-Alzola, "A theoretical framework to three-dimensional ultrasound reconstruction from irregularly sampled data," Ultrasound in Medicine & Biology, vol. 29, no. 2, pp. 255-269, Feb. 2003.
[2] T. C. Poon and R. N. Rohling, "Three-dimensional extended field-of-view ultrasound.," Ultrasound in medicine & biology, vol. 32, no. 3, pp. 357-69, Mar. 2006.
[3] C. Yao, J. M. Simpson, T. Schaeffter, and G. P. Penney, "Spatial compounding of large numbers of multi-view 3D echocardiography images using feature consistency," 2010 IEEE International Symposium on Biomedical Imaging: From Nano to Macro, pp. 968-971, 2010.
[4] A. Gee, R. Prager, G. Treece, and L. Berman, "Engineering a freehand 3D ultrasound system," Pattern Recognition Letters, vol. 24, no. 4-5, pp. 757-777, Feb. 2003.
[5] P. Toonkum, N. C. Suwanwela, and C. Chinrungrueng, "Reconstruction of 3D ultrasound images based on Cyclic Regularized Savitzky-Golay filters.," Ultrasonics, vol. 51, no. 2, pp. 136-47, Feb. 2011.
[6] C. O. Laura, K. Drechsler, M. Erdt, M. Keil, M. Noll, S. D. Beni, G. Sakas, and L. Solbiati, "for Liver Tumor Ablation," pp. 133-140, 2012.
[7] R. Rohling, a Gee, and L. Berman, "A comparison of freehand three-dimensional ultrasound reconstruction techniques.," Medical image analysis, vol. 3, no. 4, pp. 339-59, Dec. 1999.
[8] P. Hellier, N. Azzabou, and C. Barillot, "3D Freehand Ultrasound Reconstruction," pp. 597-604, 2005.
[9] C.-H. Lin, C.-M. Weng, and Y.-N. Sun, "Ultrasound image compounding based on motion compensation.," Conference proceedings: ... Annual International Conference of the IEEE Engineering in Medicine and Biology Society. IEEE Engineering in Medicine and Biology Society. Conference, vol. 6, pp. 6445-8, Jan. 2005.
[10] a Fenster and D. B. Downey, "Three-dimensional ultrasound imaging.," Annual review of biomedical engineering, vol. 2, pp. 457-75, Jan. 2000.
[11] L. Mercier, T. Langø, F. Lindseth, and L. D. Collins, "A review of calibration techniques for freehand 3-D ultrasound systems.," Ultrasound in medicine & biology, vol. 31, no. 2, pp. 143-65, Feb. 2005.
[12] O. V. Solberg, F. Lindseth, H. Torp, R. E. Blake, and T. a Nagelhus Hernes, "Freehand 3D ultrasound reconstruction algorithms--a review.," Ultrasound in medicine & biology, vol. 33, no. 7, pp. 991-1009, Jul. 2007.
[13] S. T. M. Eairs, J. E. N. S. B. Eyer, and M. I. H. Ennerici, "Original Contribution RECONSTRUCTION AND VISUALIZATION OF IRREGULARLY SAMPLED THREE- AND FOUR-DIMENSIONAL ULTRASOUND DATA FOR CEREBROVASCULAR APPLICATIONS," vol. 26, no. 2, pp. 263-272, 2000.
[14] C. O. Laura, K. Drechsler, M. Erdt, M. Keil, M. Noll, S. D. Beni, G. Sakas, and L. Solbiati, "for Liver Tumor Ablation," pp. 133-140, 2012.
[15] C. Ag, "Image guided liver surgery," International Journal of Computer Assisted Radiology and Surgery, vol. 7, no. S1, pp. 141-145, May 2012.
[16] J. A. Noble and D. Boukerroui, "Ultrasound image segmentation: a survey.," IEEE transactions on medical imaging, vol. 25, no. 8, pp. 987-1010, Aug. 2006.

## Claims

1. A Method for 3D ultrasound image acquisition and registration of a 3D model to a pre-acquired 3D image, comprising the steps of:
- providing a pre-acquired 3D image (305) of an object (110),
- displaying said pre-acquired image (305) on a display (101),
- selecting a volume of interest (301) of the object (110) in said pre-acquired image (305),
- when the spatial position of the volume of interest (301) is selected or adjusted as intended: triggering the acquisition of ultrasound images (401, 402) in said volume of interest (301) in order to generate a 3D model (403) of said object (110) in said volume of interest (301), and
- acquiring a plurality of ultrasound images (401, 402) for generating said 3D model (403) by means of said ultrasound probe (103) in the volume of interest (301) while moving the ultrasound probe (103) with respect to said object (110) along the volume of interest (301),
**characterized in that** the method further comprises the steps of:
- checking using a data processing system (105) if the current ultrasound image (401, 402) has at least a pixel in the volume of interest (301), wherein in case the current image (401, 402) has no pixel in the volume of interest (301), the current image (401, 402) is discarded by the data processing system (105), wherein otherwise the current ultrasound image (401, 402) is segmented and compounded by the data processing system (105) into said 3D model (403) to be generated, and
- determining using the data processing system (105) a quality measure for the 3D model (403) to be generated upon said acquisition of said ultrasound images (401, 402), wherein said acquisition of said ultrasound images (401, 402) is ended by the data processing system (105) once said quality measure is fulfilled or has reached a pre-defined level,
- registering the generated 3D model (403) using the data processing system (105) to the pre-acquired 3D image (305).

2. The method according to claim 1, wherein said quality measure is a criterion based on patient-specific data from said pre-acquired 3D image.

3. The method according to one of the preceding claims, wherein particularly said quality measure is at least one of:
- the number of single ultrasound images (401, 402) scanned within the volume of interest (301),
- the density of the acquired ultrasound images (401, 402) within the volume of interest (301),
- the number and/or distribution of image features, particularly the number of segmented anatomic structures in the volume of interest (301),
- the time needed for scanning the ultrasound images (401, 402),

4. The method according to one of the preceding claims, wherein a user acquiring said plurality of ultrasound images is guided to move the ultrasound probe (103) to a location where image features are expected based on the pre-acquired 3D image, particularly so as to provide a sufficient dataset for registering the generated 3D model to the pre-acquired 3D image (305).

5. The method according to one of the preceding claims, wherein an initial registration is performed, particularly in order to correctly display the position of the volume of interest (301), the acquired current ultrasound image (401, 402), and/or the 3D model (403) with respect to the pre-acquired image (305) on the display (101), wherein the initial registration involves the steps of: selecting a point in the coordinate system of the pre-acquired image (305), calculating an expected ultrasound image at this location, acquiring a corresponding ultrasound image (401, 402) of the object (110) with the ultrasound probe (103) being tracked in the room-fixed or patient-fixed coordinate system of the ultrasound probe (103), and determining a registration transform between said coordinate systems using the expected ultrasound image and the acquired ultrasound image (401, 402).

6. The method according to one of the preceding claims, wherein the current image (401, 402) is displayed in real-time on said display (101), wherein particularly the current image is displayed two-dimensionally (402) on said display and/or three-dimensionally (401), wherein said three-dimensionally displayed current ultrasound image is particularly overlaid on the displayed pre-acquired image (305), or wherein the contents of the pre-acquired 3D image are particularly overlaid on the current two-dimensional image.

7. The method according to one of the preceding claims, wherein the 3D model is displayed in real-time on the display (101) and particularly overlaid on the displayed pre-acquired image (305), wherein particularly in case a new current ultrasound image (401, 402) is compounded into the 3D model (403), the displayed 3D model (403) on the display (101) is updated.

8. The method according to one of the preceding claims, wherein an artefact detection is conducted for the non-discarded current ultrasound image (401, 402), wherein particularly in case an artefact is detected in the current ultrasound image this current ultrasound image is discarded, and wherein particularly an artefact probability is calculated based on patient-specific features of the pre-acquired 3D image.

9. The method according to one of the preceding claims, wherein said segmentation of the individual current ultrasound image (401, 402) is conducted using at least one algorithm providing segmentation of specific anatomic structures of the object in the volume of interest, particularly vessels, tumors, organ boundaries, bile ducts, and/or other anatomy, wherein particularly said algorithm is selected depending on patient-specific features of the pre-acquired 3D image.

10. Method according to one of the preceding claims, wherein guiding information is displayed on said display (101) and/or acoustically provided to the user (100), particularly verbally, in order to assist and/or guide the user (100) concerning positioning and/or moving of the ultrasound probe (103), wherein particularly said guiding information is provided through feedback based on said pre-acquired 3D image and acquired features of the 3D model.

11. The method according to one of the preceding claims, wherein after selecting a volume of interest (301) of the object (110) in said pre-acquired image (305) the spatial position of said volume of interest (301) with respect to said pre-acquired image (305) is adjusted by positioning an ultrasound probe (103) with respect to the object (110) accordingly.

12. The method according to one of the preceding claims, wherein the current spatial position of said volume of interest (301) on said display (101) with respect to said pre-acquired image (305) is visualized, particularly in real-time.

13. The method according to claim 12, wherein the visualization of the volume of interest (301) is overlaid on the displayed pre-acquired image (305).

14. The method according to one of the preceding claims, wherein particularly upon said segmentation, missing information in the current ultrasound image (401, 402) is interpolated using a-priori information about the object (110) or particularly patient-specific features from the pre-acquired 3D image.

15. The method according to one of the preceding claims, wherein upon said segmentation, missing information in the current ultrasound image (401, 402) is interpolated using cohort specific and/or statistical information about the distribution of vascular structures, geometric shapes of the anatomic structures of interest in the object, object parts or lesions, and/or other known anatomical structures.

## Patentansprüche

1. Ein Verfahren zur 3D-Ultraschallbildaufnahme und Registrierung eines 3D-Modells zu einem zuvor aufgenommenen 3D-Bild, das die folgenden Schritte aufweist:
- Bereitstellen eines zuvor aufgenommenen 3D-Bildes (305) eines Objekts (110),
- Darstellen des zuvor aufgenommenen Bildes (305) auf einem Display (101),
- Auswählen eines Volumens von Interesse (301) des Objekts (110) in dem zuvor aufgenommenen Bild (305),
- wenn die räumliche Position des Volumens von Interesse (301) wie beabsichtigt ausgewählt oder angepasst ist: Auslösen der Aufnahme von Ultraschallbildern (401, 402) in dem Volumen von Interesse (301), um ein 3D-Modell (403) des Objekts (110) in dem Volumen von Interesse (301) zu erzeugen, und
- Aufnehmen einer Vielzahl von Ultraschallbildern (401, 402) zum Erzeugen des 3D-Modells (403) mittels der Ultraschallsonde (103) in dem Volumen von Interesse (301), während die Ultraschallsonde (103) in Bezug auf das Objekt (110) entlang des Volumens von Interesse (301) bewegt wird,
**dadurch gekennzeichnet, dass** das Verfahren ferner die folgenden Schritte aufweist:
- Prüfen unter Verwendung eines Datenverarbeitungssystems (105), ob das aktuelle Ultraschallbild (401, 402) mindestens ein Pixel in dem Volumen von Interesse (301) aufweist, wobei für den Fall, dass das aktuelle Bild (401, 402) kein Pixel in dem Volumen von Interesse (301) aufweist, das aktuelle Bild (401, 402) von dem Datenverarbeitungssystem (105) verworfen wird, wobei andernfalls das aktuelle Ultraschallbild (401, 402) von dem Datenverarbeitungssystem (105) segmentiert und zu dem zu erzeugenden 3D-Modell (403) hinzugenommen wird, und
- Bestimmen unter Verwendung des Datenverarbeitungssystems (105) eines Qualitätsmaßes für das zu erzeugende 3D-Modell (403) bei der Aufnahme der Ultraschallbilder (401, 402), wobei die Aufnahme der Ultraschallbilder (401, 402) durch das Datenverarbeitungssystem (105) beendet wird, sobald das Qualitätsmaß erfüllt ist oder ein vordefiniertes Niveau erreicht hat,
- Registrieren des erzeugten 3D-Modells (403) unter Verwendung des Datenverarbeitungssystems (105) mit dem zuvor aufgenommenen 3D-Bild (305).

2. Das Verfahren nach Anspruch 1, wobei das Qualitätsmaß ein Kriterium ist, das auf patientenspezifischen Daten aus dem zuvor aufgenommenen 3D-Bild basiert.

3. Das Verfahren nach einem der vorhergehenden Ansprüche, wobei insbesondere das Qualitätsmaß mindestens eines ist von:
- der Anzahl der einzelnen Ultraschallbilder (401, 402), die innerhalb des Volumens von Interesse (301) aufgenommen wurden,
- der Dichte der aufgenommenen Ultraschallbilder (401, 402) innerhalb des Volumens von Interesse (301),
- die Anzahl und/oder Verteilung von Bildmerkmalen, insbesondere die Anzahl der segmentierten anatomischen Strukturen im Volumen von Interesse (301),
- die für die Aufnahme der Ultraschallbilder (401, 402) benötigte Zeit,

4. Das Verfahren nach einem der vorhergehenden Ansprüche, wobei ein Benutzer, der die Vielzahl von Ultraschallbildern aufnimmt, dazu angeleitet wird, die Ultraschallsonde (103) an eine Stelle zu bewegen, an der Bildmerkmale auf der Grundlage des zuvor aufgenommenen 3D-Bildes erwartet werden, insbesondere um einen ausreichenden Datensatz für die Registrierung des erzeugten 3D-Modells mit dem zuvor aufgenommenen 3D-Bild (305) bereitzustellen.

5. Das Verfahren nach einem der vorhergehenden Ansprüche, wobei eine anfängliche Registrierung durchgeführt wird, insbesondere um die Position des Volumens von Interesse (301), des aufgenommenen aktuellen Ultraschallbildes (401, 402) und/oder des 3D-Modells (403) in Bezug auf das zuvor aufgenommene Bild (305) auf dem Display (101) korrekt darzustellen, wobei die anfängliche Registrierung die Schritte beinhaltet: Auswählen eines Punktes im Koordinatensystem des zuvor aufgenommenen Bildes (305), Berechnen eines erwarteten Ultraschallbildes an dieser Stelle, Aufnehmen eines entsprechenden Ultraschallbildes (401, 402) des Objekts (110), wobei die Ultraschallsonde (103), in dem raumfesten oder patientenfesten Koordinatensystem der Ultraschallsonde (103) verfolgt wird, und Bestimmen einer Registrierungstransformation zwischen den Koordinatensystemen unter Verwendung des erwarteten Ultraschallbildes und des aufgenommenen Ultraschallbildes (401, 402).

6. Das Verfahren nach einem der vorhergehenden Ansprüche, wobei das aktuelle Bild (401, 402) in Echtzeit auf dem Display (101) dargestellt wird, wobei insbesondere das aktuelle Bild zweidimensional (402) auf dem Display und/oder dreidimensional (401) dargestellt wird, wobei das dreidimensional dargestellte aktuelle Ultraschallbild insbesondere mit dem dargestellten zuvor aufgenommenen Bild (305) überlagert wird, oder wobei der Inhalt des zuvor aufgenommenen 3D-Bildes insbesondere mit dem aktuellen zweidimensionalen Bild überlagert wird.

7. Das Verfahren nach einem der vorhergehenden Ansprüche, wobei das 3D-Modell in Echtzeit auf dem Display (101) angezeigt und insbesondere mit dem angezeigten zuvor aufgenommenen Bild (305) überlagert wird, wobei insbesondere bei einem Hinzunehmen eines neuen aktuellen Ultraschallbildes (401, 402) zu dem 3D-Modell (403) das angezeigte 3D-Modell (403) auf dem Display (101) aktualisiert wird.

8. Das Verfahren nach einem der vorhergehenden Ansprüche, wobei für das nicht verworfene aktuelle Ultraschallbild (401, 402) eine Artefaktdetektion durchgeführt wird, wobei insbesondere im Falle der Detektion eines Artefakts im aktuellen Ultraschallbild dieses aktuelle Ultraschallbild verworfen wird, und wobei insbesondere eine Artefaktwahrscheinlichkeit anhand von patientenspezifischen Merkmalen des zuvor aufgenommenen 3D-Bildes berechnet wird.

9. Das Verfahren nach einem der vorhergehenden Ansprüche, wobei die Segmentierung des einzelnen aktuellen Ultraschallbildes (401, 402) unter Verwendung mindestens eines Algorithmus durchgeführt wird, der eine Segmentierung spezifischer anatomischer Strukturen des Objekts im Volumen von Interesse, insbesondere von Gefäßen, Tumoren, Organgrenzen, Gallengängen und/oder anderer Anatomie, bereitstellt, wobei insbesondere der Algorithmus in Abhängigkeit von patientenspezifischen Merkmalen des zuvor aufgenommenen 3D-Bildes ausgewählt wird.

10. Das Verfahren nach einem der vorhergehenden Ansprüche, wobei Führungsinformationen auf dem Display (101) angezeigt und/oder dem Benutzer (100) akustisch, insbesondere verbal, zur Verfügung gestellt werden, um den Benutzer (100) bei der Positionierung und/oder Bewegung der Ultraschallsonde (103) zu unterstützen und/oder zu führen, wobei insbesondere die Führungsinformationen durch Rückkopplung basierend auf dem zuvor aufgenommenen 3D-Bild und erfassten Merkmalen des 3D-Modells bereitgestellt werden.

11. Das Verfahren nach einem der vorhergehenden Ansprüche, wobei nach Auswahl eines Volumens von Interesse (301) des Objekts (110) in dem zuvor aufgenommenen Bild (305) die räumliche Position des Volumens von Interesse (301) in Bezug auf das zuvor aufgenommene Bild (305) durch entsprechende Positionierung einer Ultraschallsonde (103) in Bezug auf das Objekt (110) angepasst wird.

12. Das Verfahren nach einem der vorhergehenden Ansprüche, wobei die aktuelle räumliche Position des Volumens von Interesse (301) auf dem Display (101) in Bezug auf das zuvor aufgenommene Bild (305) visualisiert wird, insbesondere in Echtzeit.

13. Das Verfahren nach Anspruch 12, wobei die Visualisierung des Volumens von Interesse (301) mit dem angezeigten zuvor aufgenommenen Bild (305) überlagert wird.

14. Das Verfahren nach einem der vorhergehenden Ansprüche, wobei insbesondere bei der Segmentierung fehlende Informationen im aktuellen Ultraschallbild (401, 402) unter Verwendung von a-priori-Informationen über das Objekt (110) oder insbesondere patientenspezifischen Merkmalen aus dem zuvor aufgenommenen 3D-Bild interpoliert werden.

15. Das Verfahren nach einem der vorhergehenden Ansprüche, wobei bei der Segmentierung fehlende Informationen im aktuellen Ultraschallbild (401, 402) unter Verwendung von kohortenspezifischen und/oder statistischen Informationen über die Verteilung von Gefäßstrukturen, geometrischen Formen der anatomischen Strukturen von Interesse im Objekt, Objektteilen oder Läsionen und/oder anderen bekannten anatomischen Strukturen interpoliert werden.

## Revendications

1. Procédé pour l'acquisition d'images ultrasonores 3D et l'enregistrement d'un modèle 3D dans une image 3D pré-acquise, comportant les étapes de :
- fourniture d'une image 3D pré-acquise (305) d'un objet (110),
- affichage de ladite image pré-acquise (305) sur un afficheur (101),
- sélection d'un volume d'intérêt (301) de l'objet (110) dans ladite image pré-acquise (305),
- lorsque la position spatiale du volume d'intérêt (301) est sélectionnée ou ajustée comme prévu : déclenchement de l'acquisition d'images (401, 402) ultrasonores dans ledit volume d'intérêt (301) afin de générer un modèle 3D (403) dudit objet (101) dans ledit volume d'intérêt (301) et
- acquisition d'une pluralité d'images (401, 402) ultrasonores pour générer ledit modèle 3D (403) au moyen de ladite sonde ultrasonore (103) dans le volume d'intérêt (301) tout en déplaçant la sonde ultrasonore (103) par rapport audit objet (110) le long du volume d'intérêt (301),
**caractérisé en ce que** le procédé comporte en outre les étapes de:
- contrôle à l'aide d'un système de traitement de données (105) si l'image (401, 402) ultrasonore actuelle a au moins un pixel dans le volume d'intérêt (301), dans lequel, dans le cas où l'image (401, 402) actuelle n'a pas de pixel dans le volume d'intérêt (301), l'image (401, 402) actuelle est rejetée par le système de traitement de données (105), dans lequel, autrement, l'image (401, 402) ultrasonore actuelle est segmentée et composée par le système de traitement de données (105) en ledit modèle 3D (403) à générer et
- détermination, à l'aide du système de traitement de données (105), d'une mesure de qualité pour le modèle 3D (403) à générer lors de ladite acquisition desdites images (401, 402) ultrasonores, dans lequel ladite acquisition desdites images (401, 402) ultrasonores est terminée par le système de traitement de données (105) une fois que ladite mesure de qualité est satisfaite ou a atteint un niveau prédéfini,
- enregistrement du modèle 3D (403) généré à l'aide du système de traitement de données (105) dans l'image 3D pré-acquise (305).

2. Procédé selon la revendication 1, dans lequel ladite mesure de qualité est un critère basé sur des données spécifiques au patient provenant de ladite image 3D pré-acquise.

3. Procédé selon l'une des revendications précédentes, dans lequel, en particulier, ladite mesure de qualité est au moins un élément parmi :
- le nombre d'images (401, 402) ultrasonores uniques balayées au sein du volume d'intérêt (301),
- la densité des images (401, 402) ultrasonores acquises au sein du volume d'intérêt (301),
- le nombre et/ou la répartition de caractéristiques d'image, en particulier le nombre de structures anatomiques segmentées dans le volume d'intérêt (301),
- le temps nécessaire pour balayer les images (401, 402) ultrasonores,

4. Procédé selon l'une des revendications précédentes, dans lequel un utilisateur acquérant ladite pluralité d'images ultrasonores est guidé pour déplacer la sonde ultrasonore (103) vers un emplacement où des caractéristiques d'image sont attendues sur la base de l'image 3D pré-acquise, en particulier de manière à fournir un ensemble de données suffisant pour enregistrer le modèle 3D généré dans l'image 3D pré-acquise (305).

5. Procédé selon l'une des revendications précédentes, dans lequel un enregistrement initial est réalisé, en particulier afin d'afficher correctement la position du volume d'intérêt (301), l'image (401, 402) ultrasonore actuelle acquise et/ou le modèle 3D (403) par rapport à l'image pré-acquise (305) sur l'afficheur (101), dans lequel l'enregistrement initial implique les étapes de : sélection d'un point dans le système de coordonnées de l'image pré-acquise (305), calcul d'une image ultrasonore attendue à cet emplacement, acquisition d'une image (401, 402) ultrasonore correspondante de l'objet (110), la sonde ultrasonore (103) étant suivie dans le système de coordonnées fixe pour un patient ou fixe pour une salle de la sonde ultrasonore (103) et détermination d'une transformée d'enregistrement entre lesdits systèmes de coordonnées utilisant l'image ultrasonore attendue et l'image (401, 402) ultrasonore acquise.

6. Procédé selon l'une des revendications précédentes, dans lequel l'image (401, 402) actuelle est affichée en temps réel sur ledit afficheur (101), dans lequel, en particulier, l'image actuelle est affichée en deux dimensions (402) sur ledit afficheur et/ou en trois dimensions (401), dans lequel ladite image ultrasonore actuelle affichée en trois dimensions est, en particulier, superposée sur l'image pré-acquise (305) affichée ou le contenu de l'image 3D pré-acquise étant, en particulier, superposé sur l'image en deux dimensions actuelle.

7. Procédé selon l'une des revendications précédentes, dans lequel le modèle 3D est affiché en temps réel sur l'afficheur (101) et, en particulier, superposé sur l'image pré-acquise (305) affichée, dans lequel, en particulier, au cas où une nouvelle image (401, 402) ultrasonore actuelle est composée dans le modèle 3D (403), le modèle 3D affiché (403) sur l'afficheur (101) est mis à jour.

8. Procédé selon l'une des revendications précédentes, dans lequel une détection d'artefacts est menée pour l'image (401, 402) ultrasonore actuelle non rejetée, dans lequel, en particulier, au cas où un artefact est détecté dans l'image ultrasonore actuelle, cette image ultrasonore actuelle est rejetée et dans lequel, en particulier, une probabilité d'artefacts est calculée sur la base de caractéristiques spécifiques au patient de l'image 3D pré-acquise.

9. Procédé selon l'une des revendications précédentes, dans lequel ladite segmentation de l'image (401, 402) ultrasonore actuelle individuelle est menée à l'aide d'au moins un algorithme fournissant une segmentation de structures anatomiques spécifiques de l'objet dans le volume d'intérêt, en particulier des vaisseaux, des tumeurs, des limites d'organes, des conduits biliaires et/ou d'autres parties anatomiques, dans lequel, en particulier, ledit algorithme est sélectionné en fonction de caractéristiques spécifiques au patient de l'image 3D pré-acquise.

10. Procédé selon l'une des revendications précédentes, dans lequel des informations de guidage sont affichées sur ledit afficheur (101) et/ou fournies acoustiquement à l'utilisateur (100), en particulier oralement, afin d'aider et/ou de guider l'utilisateur (100) concernant le positionnement et/ou le déplacement de la sonde ultrasonore (103), dans lequel, en particulier, lesdites informations de guidage sont fournies à travers des rétroactions basées sur ladite image 3D pré-acquise et des caractéristiques acquises du modèle 3D.

11. Procédé selon l'une des revendications précédentes, dans lequel, après la sélection d'un volume d'intérêt (301) de l'objet (110) dans ladite image pré-acquise (305), la position spatiale dudit volume d'intérêt (301) par rapport à ladite image pré-acquise (305) est ajustée par positionnement d'une sonde ultrasonore (103) par rapport à l'objet (110) en conséquence.

12. Procédé selon l'une des revendications précédentes, dans lequel la position spatiale actuelle dudit volume d'intérêt (301) sur ledit afficheur (101) par rapport à ladite image pré-acquise (305) est visualisée, en particulier en temps réel.

13. Procédé selon la revendication 12, dans lequel la visualisation du volume d'intérêt (301) est superposée sur l'image pré-acquise (305) affichée.

14. Procédé selon l'une des revendications précédentes, dans lequel, en particulier, lors de ladite segmentation, des informations manquantes dans l'image (401, 402) ultrasonore actuelle sont interpolées à l'aide d'informations a priori à propos de l'objet (110) ou, en particulier, de caractéristiques spécifiques au patient provenant de l'image 3D pré-acquise.

15. Procédé selon l'une des revendications précédentes, dans lequel, lors de ladite segmentation, des informations manquantes dans l'image (401, 402) ultrasonore actuelle sont interpolées à l'aide d'informations statistiques et/ou spécifiques à une cohorte à propos de la répartition de structures vasculaires, de formes géométriques des structures anatomiques d'intérêt dans l'objet, d'autres parties ou lésions d'objet et/ou d'autres structures anatomiques connues.
